# EUROPEAN PATENT SPECIFICATION

(11) **EP 3 248 644 B1**
(45) Date of publication and mention of the grant of the patent: **12.08.2020**
(21) Application number: 16829222.5
(22) Date of filing: 10.03.2016
(51) Int. Cl.: A61M 25/09, A61B 17/12

(54) **GUIDE WIRE**
FÜHRUNGSDRAHT
FIL-GUIDE

(43) Date of publication of application: 29.11.2017
(73) Proprietor: Asahi Intecc Co., Ltd., Seto-shi, Aichi 489-0071 (JP)
(72) Inventor: FURUKAWA, Muneya, Seto-shi, Aichi 489-0071 (JP)
(74) Representative: Winter, Brandl, Fürniss, Hübner, Röss, Kaiser, Polte - Partnerschaft mbB
(86) International application number: PCT/JP2016/057539
(87) International publication number: WO 2017/154164

(56) References cited:
- EP-A1- 2 810 683
- EP-A1- 2 918 306
- JP-A- 2015 171 519
- US-A1- 2008 281 230
- US-B1- 6 251 086

## Description

### TECHNICAL FILED

The present invention relates to a guide wire used as a guide for inserting a catheter into a lumen in the body such as a blood vessel and a ureter, and for inserting an indwelling device into a site of aneurysm formation in a blood vessel.

### BACKGROUND ART

A guide wire used as a guide for inserting a catheter into a lumen in the body such as a blood vessel and a ureter, and for inserting an indwelling device into a site of an aneurysm formation in a blood vessel generally comprises a core shaft, a coil body covering the front end part of the core shaft, a front end joining portion joining the front end of the core shaft to the front end of the coil body, and a base end joining portion joining the core shaft to the base end of the coil body.

For example, US 2007/0185415 A1 describes a guide wire comprising a core wire (hereafter referred to as a core shaft), a coil body covering a front end part of the core shaft, and a front end joining portion joining the front end of the core shaft to the front end of the coil body. Patent Document 1 also describes that the guide wire has an appearance in which the front end joining portion is joined to the front end of the coil body (see, for example, FIG. 12A and FIG. 12B).

In order to insert a catheter into a patient's body, for example, an operator inserts the guide wire described in Patent Document 1 into the patient's body so that it advances toward a narrowed segment and the like in the patient's body, and then penetrates the narrowed segment. However, the guide wire may be stuck at the narrowed segment when the operator tries to penetrate the narrowed segment with the guide wire. If that happens, the operator tries to push, pull or rotate a connector fixed at the base end part of the guide wire to release the guide wire stuck there.

However, in the case of a conventional guide wire, the front end joining portion of the guide wire may be detached from the coil body when the guide wire is stuck at a narrowed segment and the like, and an operator tries to push, pull, or rotate the guide wire. If the front end joining portion is detached from the coil body, the front end joining portion alone may be left in the patient's body, resulting in a problem in that the subsequent treatment is very difficult.

EP 2 810 683 A1 may be regarded as a closest prior art and discloses a guide wire comprising a core shaft, a coil body and a joining portion, wherein the base end of the joining portion is formed into an uneven shape in the longitudinal direction of the guide wire.

### SUMMARY OF INVENTION

### TECHNICAL PROBLEM

The present invention is made in order to solve the above problem. The object of the present invention is to provide a guide wire in which the joining strength between a front end joining portion of the guide wire and a coil body is improved to prevent detachment of the front end joining portion from the coil body.

### SOLUTION TO PROBLEM

In order to achieve the above object, a guide wire according to claim 1 is provided. Further advantageous aspects are defined in the dependent claims.

### ADVANTAGEOUS EFFECTS OF INVENTION

The guide wire according to the first aspect of the present invention comprises a core shaft, a coil body covering the outer periphery of the core shaft, and a joining portion joining the front end of the core shaft to the front end of the coil body at a base end of the joining portion, in which the base end of the joining portion is formed into an uneven shape in the longitudinal direction along the circumferential direction of the joining portion, the uneven shape of the base end of the joining portion is formed of protruded portions protruded to the base end side of the guide wire and depressed portions protruded to the front end side of the guide wire, and a space is defined by the protruded portions and the core shaft. Therefore, the joining strength between the joining portion and the coil body can improve, showing an effect of preventing detachment of the joining portion from the coil body.

Moreover, according to the guide wire of the second aspect, the protruded portion protruded to the front end side in the longitudinal direction is formed in a curved fashion in the guide wire according to the first aspect. Therefore, stress concentration to the joining portion can be prevented when the guide wire is bent, showing an effect of further preventing detachment of the joining portion from the coil body, in addition to the effect of the guide wire according to the first aspect.

Furthermore, according to the guide wire of the third aspect, the coil body is formed by spirally winding two or more twisted wires each having multiple twisted element wires, with reference to the guide wire according to the first or second aspect of the present invention. Therefore, this can provide the effect of the guide wire according to the first or second aspect in a simpler manner.

### BRIEF DESCRIPTION OF DRAWINGS

Fig. 1 shows the entire view of a guide wire according to the first embodiment of the present invention.
Fig. 2 shows an enlarged view of the front end of Fig. 1.
Fig. 3 shows a longitudinal sectional view of Fig. 2.
Fig. 4 shows an enlarged view of the front end of a guide wire according to the second embodiment.
Fig. 5 shows a longitudinal sectional view of Fig. 4.
Fig. 6 shows an enlarged view of the front end of a guide wire according to the third embodiment.
Fig. 7 shows a longitudinal sectional view of Fig. 6.
Fig. 8 shows the entire view of a guide wire according to the fourth embodiment.
Fig. 9 shows an enlarged view of the front end of Fig. 8.

### DESCRIPTION OF EMBODIMENTS

Below, the aforementioned embodiments of the present invention will be described with reference to the drawings.

### First embodiment

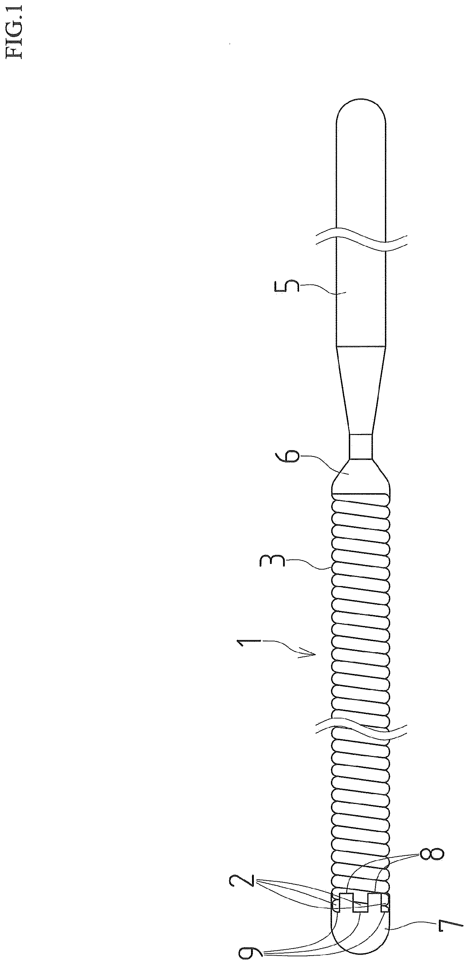

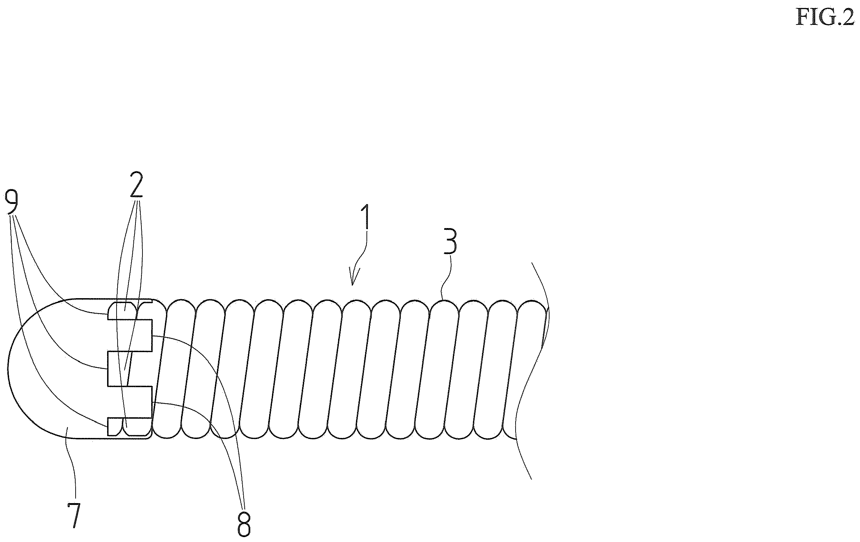

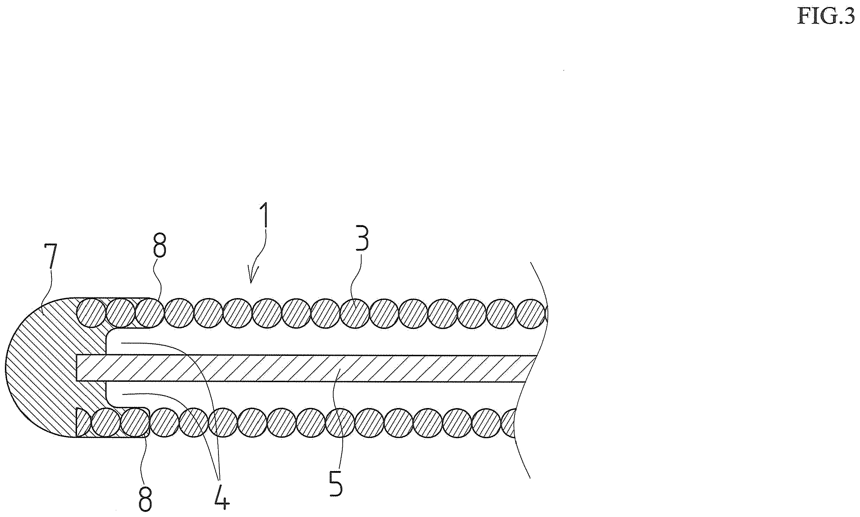

Fig. 1 shows the entire view of the guide wire according to the first embodiment of the present invention. Fig. 2 shows an enlarged view of the front end of Fig. 1. Fig. 3 shows a longitudinal sectional view of Fig. 2.

With reference to Fig.1, a guide wire 1 comprises a core shaft 5, a coil body 3 fixed to the front end part of the core shaft 5, a front end joining portion 7 joining the front end of the coil body 3 to the front end of the core shaft 5, and a base end joining portion 6 joining the base end of the coil body 3 to the core shaft 5.

The core shaft 5 has a shape of a round rod tapered toward the front end side from the base end. There is no particular limitation for the material of the core shaft 5, but stainless steel is used in the present embodiment. In addition to this, superelastic alloys such as Ni-Ti alloy may also be used.

The coil body 3, which is of a hollow cylindrical shape, is spirally wound around the core shaft 5. There is no particular limitation for the material of the coil body 3 as well, but stainless steel is used in the present embodiment. In addition to this, tungsten may also be used.

Note that the coil body 3 in the present embodiment is formed with one metal element wire such that the twist angle to the longitudinal direction of the guide wire 1 is relatively large.

The front end joining portion 7, which is of an approximately hemispherical shape, constitutes the front end of the guide wire 1, and the outer diameter of the front end joining portion 7 at the base end part is approximately the same as that of the coil body 3. There is no particular limitation for the material of the front end joining portion 7 as well, but an Ag-Sn-based solder material is used in the present embodiment. In addition to this, an Au-Sn-based solder material may also be used.

Further, the front end joining portion 7 is configured to have a rectangular protruded portion 8 protruded to the base end side in the longitudinal direction of the guide wire 1, and a rectangular depressed portion 9 protruded to the front end side in the longitudinal direction. That is, as seen at a cross-section (which corresponds to the "cross-sectional view" in the present invention) of the front end joining portion 7 cut in the direction perpendicular to the plane of the sheet of Fig. 2 (cut in the direction perpendicular to the longitudinal direction of the guide wire 1), the length of the guide wire 1 in the longitudinal direction differs along the direction of the outer periphery of the cross-section, and the rectangular protruded portion 8 protruded to the base end side in the longitudinal direction and the rectangular depressed portion 9 protruded to the front end side in the longitudinal direction form an uneven shape in the longitudinal direction.

The outer diameter of the base end joining portion 6 at the front end part is approximately the same as that of the coil body 3. There is no particular limitation for the material of the front end joining portion 6 as well, but an Ag-Sn-based solder material is used in the present embodiment. In addition to this, an Au-Sn-based solder material may also be used.

According to the guide wire 1 of the present embodiment, the base end of the front end joining portion 7 is formed into an uneven shape in the longitudinal direction along the direction of the outer periphery in a cross-sectional view, and thus a distortion of the coil body 3 upon bending the guide wire 1 can be reduced by means of a space 2 defined by the rectangular protruded portion 8 protruded to the base end side in the longitudinal direction and the rectangular depressed portion 9 protruded to the front end side in the longitudinal direction. As a result, the joining strength between the front end joining portion 7 and the coil body 3 of the guide wire 1 can be improved to prevent detachment of the front end joining portion 7 from the coil body 3.

Note that in Fig. 2, the end portion of the rectangular protruded portion 8 protruded to the base end side in the longitudinal direction does not meet along the element wire of the coil body 3. However, when the end portion of the protruded portion 8 is allowed to meet along the element wire of the coil body 3 by altering the length of the protruded portion 8, the joining strength between the front end joining portion 7 and the coil body 3 can further be improved.

Further, according to the guide wire 1 of the present embodiment, a distortion of the coil body 3 upon bending the guide wire 1 can also be reduced by means of a space 4 defined by the rectangular protruded portion 8 protruded to the base end side in the longitudinal direction and the core shaft 5 as shown in Fig. 3. As a result, the joining strength between the front end joining portion 7 and the coil body 3 can be improved to prevent detachment of the front end joining portion 7 from the coil body 3.

### Second embodiment

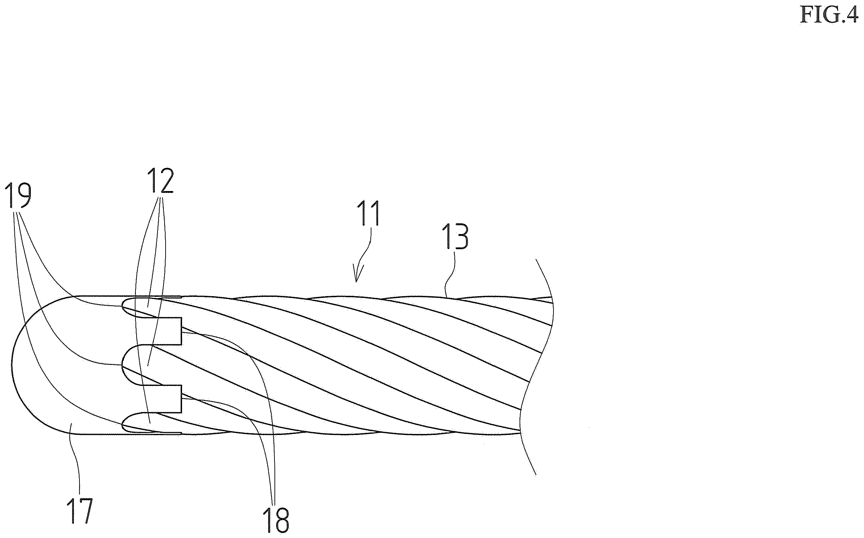

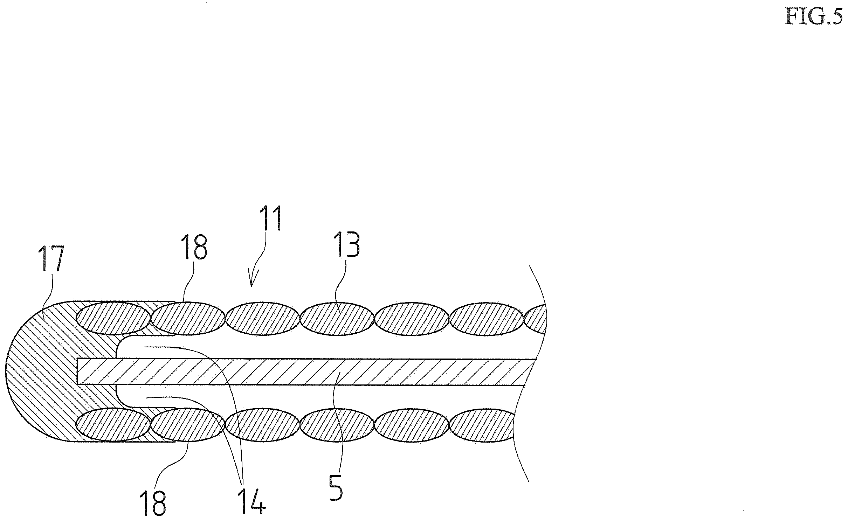

Below, a second embodiment will be described. Descriptions will be omitted for the parts in common with the first embodiment, to which the same reference numbers will be assigned in the figures. Fig. 4 shows an enlarged view of the front end of the guide wire according to the second embodiment, and Fig. 5 shows a longitudinal sectional view of Fig. 4.

With reference to Fig. 4, a guide wire 11 comprises a core shaft 5, a coil body 13 fixed to the front end part of the core shaft 5, and a front end joining portion 17 joining the front end of the coil body 13 to the front end of the core shaft 5.

The coil body 13, which is of a hollow cylindrical shape, is spirally wound around the core shaft 5. There is no particular limitation for the material of the coil body 13, but stainless steel is used in the present embodiment. In addition to this, tungsten may also be used.

Note that the coil body 13 according to the present embodiment is formed by twisting multiple metal element wires (10 metal element wires in the present embodiment) into a hollow cylindrical shape such that the twist angle to the longitudinal direction of the guide wire is relatively small.

The front end joining portion 17, which is of an approximately hemispherical shape, constitutes the front end of the guide wire 11. There is no particular limitation for the material of the front end joining portion 17 as well, but an Au-Sn-based solder material is used in the present embodiment. In addition to this, an Au-Sn-based solder material may also be used.

Further, the front end joining portion 17 is configured to have a rectangular protruded portion 18 protruded to the base end side in the longitudinal direction of the guide wire 11, and a curved depressed portion 19 protruded to the front end side in the longitudinal direction. That is, as seen at a cross-section (which corresponds to the "cross-sectional view" in the present invention) of the front end joining portion 17 cut in the direction perpendicular to the plane of the sheet of Fig. 4 (cut in the direction perpendicular to the longitudinal direction of the guide wire 11), the length of the guide wire 11 in the longitudinal direction differs along the direction of the outer periphery of the cross-section, and the rectangular protruded portion 8 protruded to the base end side in the longitudinal direction and the curved depressed portion 19 protruded to the front end side in the longitudinal direction form an uneven shape in the longitudinal direction.

According to the guide wire 11 of the present embodiment, the base end of the front end joining portion 17 is formed into an uneven shape in the longitudinal direction along the direction of the outer periphery in a cross-sectional view, and thus a distortion of the coil body 13 upon bending the guide wire 11 can be reduced by means of a space 12 defined by the rectangular protruded portion 18 protruded to the base end side in the longitudinal direction and the curved depressed portion 19 protruded to the front end side in the longitudinal direction. In addition, stress concentration to the joining portion 17 can also be prevented when the guide wire 11 is bent, further preventing detachment of the joining portion 17 from the coil body 13.

Further, according to the guide wire 11 of the present embodiment, a distortion of the coil body 13 upon bending the guide wire 11 can also be reduced by means of a space 14 defined by the rectangular protruded portion 18 protruded to the base end side in the longitudinal direction and the core shaft 5. As a result, the joining strength between the front end joining portion 17 and the coil body 13 can be improved to prevent detachment of the front end joining portion 17 from the coil body 13.

### Third embodiment

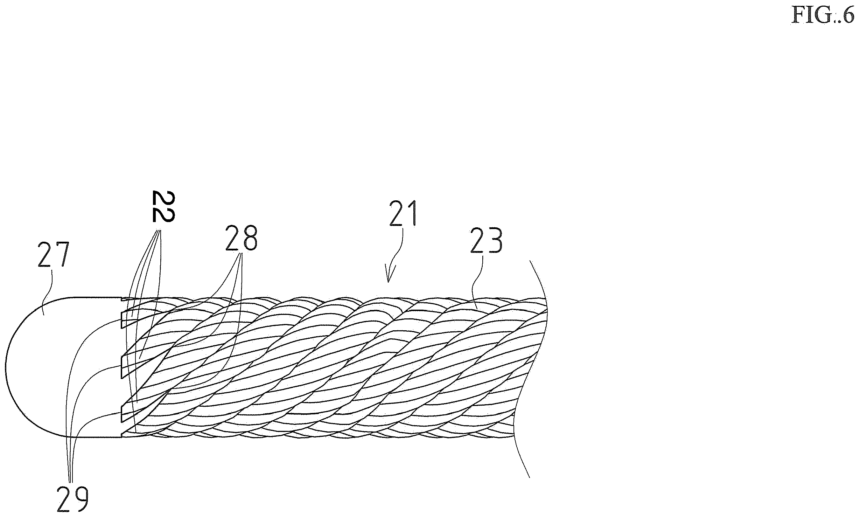

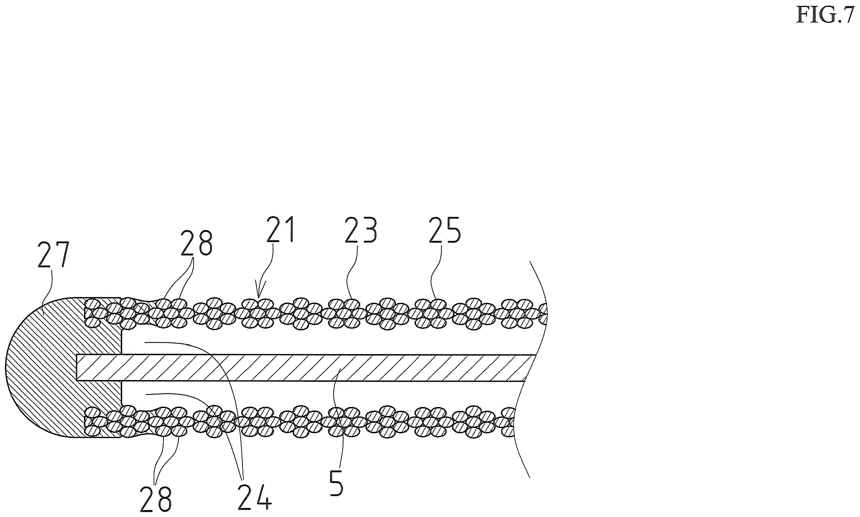

Below, a third embodiment will be described. Descriptions will be omitted for the parts in common with the first embodiment, to which the same reference numbers will be assigned in the figures. Fig. 6 shows an enlarged view of the front end of the guide wire according to the third embodiment, and Fig. 7 shows a longitudinal sectional view of Fig. 6.

With reference to Fig. 6, a guide wire 21 comprises a core shaft 5, a coil body 23 fixed to the front end part of the core shaft 5, and a front end joining portion 27 joining the front end of the coil body 23 to the front end of the core shaft 5.

The coil body 23, which is of a hollow cylindrical shape, is spirally wound around the core shaft 5. There is no particular limitation for the material of the coil body 23 as well, but stainless steel is used in the present embodiment. In addition to this, tungsten may also be used.

Note that the coil body 23 according to the present embodiment is formed by spirally winding 10 twisted wires 25 around the core shaft 5, the twisted wires 25 each being formed by twisting 7 metal element wires (see Fig. 7).

The front end joining portion 27, which is of an approximately hemispherical shape, constitutes the front end of the guide wire 21. There is no particular limitation for the material of the front end joining portion 27 as well, but an Ag-Sn-based solder material is used in the present embodiment. In addition to this, an Au-Sn-based solder material may also be used.

Further, the front end joining portion 27 is configured to have a wedge-shaped protruded portion 28 protruded to the base end side in the longitudinal direction of the guide wire 21, and a rectangular depressed portion 29 protruded to the front end side in the longitudinal direction. That is, as seen at a cross-section (which corresponds to the "cross-sectional view" in the present invention) of the front end joining portion 27 cut in the direction perpendicular to the plane of the sheet of Fig. 6 (cut in the direction perpendicular to the longitudinal direction of the guide wire 21), the length of the guide wire 21 in the longitudinal direction differs along the direction of the outer periphery of the cross-section, and the wedge-shaped protruded portion 28 protruded to the base end side in the longitudinal direction and the rectangular depressed portion 29 protruded to the front end side in the longitudinal direction form an uneven shape in the longitudinal direction.

According to the guide wire 21 of the present embodiment, the base end of the front end joining portion 27 is formed into an uneven shape in the longitudinal direction along the direction of the outer periphery in a cross-sectional view, and thus a distortion of the coil body 23 upon bending the guide wire 21 can be reduced by means of a space 22 defined by the wedge-shaped protruded portion 28 protruded to the base end side in the longitudinal direction and the rectangular depressed portion 29 protruded to the front end side in the longitudinal direction. In addition, a solder material constituting the front end joining portion 27 is allowed to permeate among the metal element wires of the twisted wires 25 to improve the joining strength between the front end joining portion 27 and the coil body 23 of the guide wire 21. This can further prevent detachment of the front end joining portion 27 from the coil body 23.

Further, according to the guide wire 21 of the present embodiment, a distortion of the coil body 23 upon bending the guide wire 21 can also be reduced by means of a space 24 defined by the wedge-shaped protruded portion 28 protruded to the base end side in the longitudinal direction and the core shaft 5 as shown in Fig. 7. As a result, the joining strength between the front end joining portion 27 and the coil body 23 can be improved to prevent detachment of the front end joining portion 27 from the coil body 23.

### Fourth embodiment

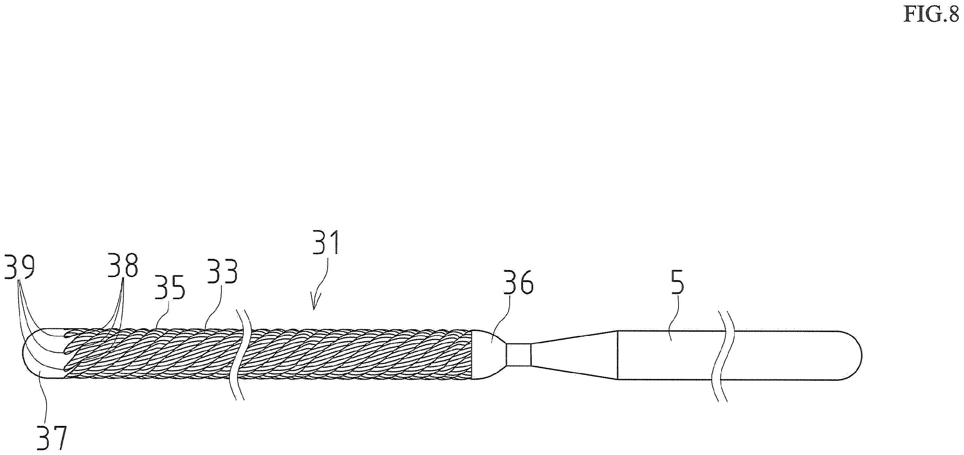

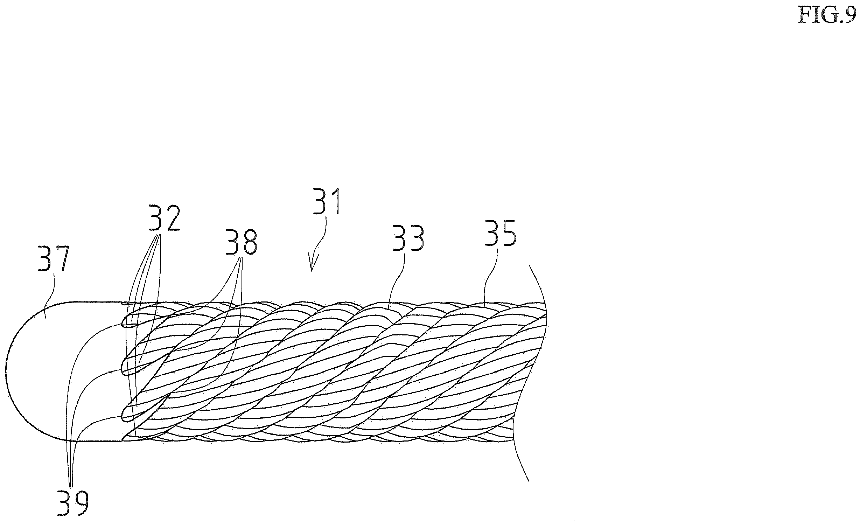

Below, a fourth embodiment will be described. Descriptions will be omitted for the parts in common with the first embodiment, to which the same reference numbers will be assigned in the figures. Fig. 8 shows the entire view of a guide wire according to the fourth embodiment of the present invention. Fig. 9 shows an enlarged view of the front end of Fig. 8. Note that a longitudinal sectional view for the present embodiment is omitted because it is the same as Fig.7.

With reference to Fig. 8, a guide wire 31 comprises a core shaft 5, a coil body 33 fixed to the front end part of the core shaft 5, the front end joining portion 37 joining the front end of the coil body 33 to the front end of the core shaft 5, and a base end joining portion 36 joining the base end of the coil body 33 to the core shaft 5.

The coil body 33, which is of a hollow cylindrical shape, is spirally wound around the core shaft 5. There is no particular limitation for the material of the coil body 33 as well, but stainless steel is used in the present embodiment. In addition to this, tungsten may also be used.

Note that the coil body 33 in the present embodiment is formed by spirally winding 10 twisted wires 35 around the core shaft 5, the twisted wires 35 each being formed by twisting 7 metal element wires as in the coil body 23 according to the third embodiment.

The front end joining portion 37, which is of an approximately hemispherical shape, constitutes the front end of the guide wire 31. There is no particular limitation for the material of the front end joining portion 37 as well, but an Ag-Sn-based solder material is used in the present embodiment. In addition to this, an Au-Sn-based solder material may also be used.

Further, the front end joining portion 37 is configured to have a wedge-like protruded portion 38 protruded to the base end side in the longitudinal direction of the guide wire 31, and a curved depressed portion 39 protruded to the front end side in the longitudinal direction. That is, as seen at a cross-section (which corresponds to the "cross-sectional view" in the present invention) of the front end joining portion 37 cut in the direction perpendicular to the plane of the sheet of Fig. 9 (cut in the direction perpendicular to the longitudinal direction of the guide wire 31), the length of the guide wire 31 in the longitudinal direction differs along the direction of the outer periphery of the cross-section, and the wedge-shaped protruded portion 38 protruded to the base end side in the longitudinal direction and the curved depressed portion 39 protruded to the front end side in the longitudinal direction form an uneven shape in the longitudinal direction.

The outer diameter of the base end joining portion 36 at the front end part is approximately the same as that of the coil body 33. There is no particular limitation for the material of the front end joining portion 36 as well, but an Ag-Sn-based solder material is used in the present embodiment. In addition to this, an Au-Sn-based solder material may also be used.

According to the guide wire 31 of the present embodiment, the base end of the front end joining portion 37 is formed into an uneven shape in the longitudinal direction along the direction of the outer periphery in the longitudinal sectional view, and thus a distortion of the coil body 33 upon bending the guide wire 31 can be reduced by means of a space 32 defined by the wedge-shaped protruded portion 38 protruded to the base end side in the longitudinal direction, and the curved depressed portion 39 protruded to the front end side in the longitudinal direction.

Further, a solder material constituting the front end joining portion 37 is allowed to permeate among the metal element wires of the twisted wires 35 to further improve the joining strength between the front end joining portion 37 and the coil body 33 of the guide wire 31. In addition, stress concentration to the joining portion 37 can be prevented when the guide wire 11 is bent, further preventing detachment of the joining portion 37 from the coil body 33.

Although the guide wires according to the various embodiments of the present invention are described above, the present invention shall not be limited to these embodiments. The present invention may be practiced with various modifications made without departing from the scope of the present invention.

For example, the coil bodies of the guide wires according to the above embodiments are formed with metal element wires, but they may be formed with one or more resin element wires. However, a coil body formed with one or more metal element wires may be convenient when the front end joining portion comprises a solder material.

Further, the front end joining portions in the aforementioned embodiments are made of an Ag-Sn-based solder material or an Au-Sn-based solder material, but a core shaft may be also fixed to a coil body with an adhesive. However, an Ag-Sn-based solder material or an Au-Sn-based solder material can provide a better bonding strength based on previous experiences.

### DESCRIPTION OF SYMBOLS

1, 11, 21, 31 Guide wire
2, 4, 12, 14, 22, 24, 32 Space
3, 13, 23, 33 Coil body
5 Core Shaft
6, 36 Base end joining portion
7, 17, 27, 37 Front end joining portion
8, 18, 28, 38 Protruded portion
9, 19, 29, 39 Depressed portion
25, 35 Twisted wire

## Claims

1. A guide wire having a longitudinal direction and having a front end side and a base end side in the longitudinal direction, the guide wire comprising:
a core shaft (5),
a coil body (3, 13, 23, 33) covering an outer periphery of the core shaft (5), and a joining portion (7, 17, 27, 37) joining a front end of the core shaft (5) to a front end of the coil body (3, 13, 23, 33) at a base end of the joining portion (7, 17, 27, 37), **characterized in that**
the base end of the joining portion (7, 17, 27, 37) is formed into an uneven shape in the longitudinal direction along the circumferential direction of the joining portion (7, 17, 27, 37) in a cross-sectional view, the uneven shape of the base end of the joining portion (7, 17, 27, 37) is formed of protruded portions (8, 18, 28, 38) protruded to the base end side of the guide wire (1, 11, 21, 31) and depressed portions (9, 19, 29, 39) protruded to the front end side of the guide wire (1, 11, 21, 31), and
a space (4, 14, 24) is defined by the protruded portions (8, 18, 28, 38) and the core shaft (5).

2. The guide wire according to claim 1, wherein the depressed portion (19, 39) protruded to the front end side is formed into a curved shape.

3. The guide wire according to claim 1 or 2, wherein the coil body (23, 33) is formed by spirally winding multiple twisted wires each having multiple twisted element wires.

4. The guide wire according to claim 1, wherein the protruded portion (28, 38) is wedge-shaped.

## Patentansprüche

1. Führungsdraht mit einer Längsrichtung und in Längsrichtung mit einer vorderen Endseite und einer Basisendseite, wobei der Führungsdraht umfasst:
eine Kernstange (5),
einen Spulenkörper (3, 13, 23, 33), der einen Außenumfang der Kernstange (5) abdeckt, und
einen Verbindungsabschnitt (7, 17, 27, 37), der an einem Basisende des Verbindungsabschnitts (7, 17, 27, 37) ein vorderes Ende der Kernstange (5) mit einem vorderen Ende des Spulenkörpers (3, 13, 23, 33) verbindet, **dadurch gekennzeichnet, dass**
das Basisende des Verbindungsabschnitts (7, 17, 27, 37) in einer Schnittansicht in einer unregelmäßigen Form in Längsrichtung entlang der Umfangsrichtung des Verbindungsabschnitts (7, 17, 27, 37) ausgebildet ist, wobei die unregelmäßige Form des Basisendes des Verbindungsabschnitts (7, 17, 27, 37) aus Vorstehabschnitten (8, 18, 28, 38), die zur Basisendseite des Führungsdrahts (1, 11, 21, 31) vorstehen, und Vertiefungsabschnitten (9, 19, 29, 39), die zur vorderen Endseite des Führungsdrahts (1, 11, 21, 31) vorstehen, ausgebildet ist, und
durch die Vorstehabschnitte (8, 18, 28, 38) und die Kernstange (5) ein Raum (4, 14, 24) definiert ist.

2. Führungsdraht nach Anspruch 1, wobei der zur vorderen Endseite vorstehende Vertiefungsabschnitt (19, 39) in einer gebogenen Form ausgebildet ist.

3. Führungsdraht nach Anspruch 1 oder 2, wobei der Spulenkörper (23, 33) durch spiralförmiges Wickeln mehrerer verdrillter Drähte, die jeweils mehrere verdrillte Elementdrähte aufweisen, ausgebildet ist.

4. Führungsdraht nach Anspruch 1, wobei der Vorstehabschnitt (28, 38) keilförmig ist.

## Revendications

1. Fil-guide présentant une direction longitudinale et présentant un côté d'extrémité avant et un côté d'extrémité de base dans la direction longitudinale, le fil-guide comprenant :
une tige centrale (5),
un corps de bobine (3, 13, 23, 33) recouvrant une périphérie extérieure de la tige centrale (5), et une partie de jonction (7, 17, 27, 37) reliant une extrémité avant de la tige centrale (5) et une extrémité avant du corps de bobine (3, 13, 23, 33) au niveau d'une extrémité de base de la partie de jonction (7, 17, 27, 37), **caractérisé en ce que**
l'extrémité de base de la partie de jonction (7, 17, 27, 37) est formée en une forme irrégulière dans la direction longitudinale le long de la direction circonférentielle de la partie de jonction (7, 17, 27, 37) selon une vue en section transversale, la forme irrégulière de l'extrémité de base de la partie de jonction (7, 17, 27, 37) est formée de parties saillantes (8, 18, 28, 38) faisant saillie vers le côté d'extrémité de base du fil-guide (1, 11, 21, 31) et de parties enfoncées (9, 19, 29, 39) faisant saillie vers le côté d'extrémité avant du fil-guide (1, 11, 21, 31), et
un espace (4, 14, 24) est défini par les parties saillantes (8, 18, 28, 38) et la tige centrale (5).

2. Fil-guide selon la revendication 1, dans lequel la partie enfoncée (19, 39) faisant saillie vers le côté d'extrémité avant est formée en une forme incurvée.

3. Fil-guide selon la revendication 1 ou 2, dans lequel le corps de bobine (23, 33) est formé en enroulant en spirale plusieurs fils torsadés présentant chacun plusieurs fils d'éléments torsadés.

4. Fil-guide selon la revendication 1, dans lequel la partie saillante (28, 38) est en forme de coin.
